# EUROPEAN PATENT APPLICATION

(11) **EP 2 243 476 A1**
(43) Date of publication of application: **27.10.2010**
(21) Application number: 09290288.1
(22) Date of filing: 17.04.2009
(51) Int. Cl.: A61K 31/14, A61K 31/155, A61K 31/352, A61K 31/4164, A61K 31/4425, C07D 311/00, A61P 25/00, A61P 25/16, A61P 25/28

(54) **Compounds for the treatment of mitochondrial diseases**

(71) Applicant: Centre National de la Recherche Scientifique, 75016 Paris (FR)
(72) Inventor: Blondel, Marc, 29250 Saint Pol de Léon (FR); Couplan, Elodie, 29950 Bénodet (FR); Di Rago, Jean-Paul, 33480 SAINTE-HELENE (FR); Dauzonne, Daniel, 75011 Paris (FR); Palladino,Michael, Pittsburg PA 15260 (US); Celotto, Alicia, Pittsburg PA 15260 (US)
(74) Representative: Bernstein, Claire Jacqueline

(57) **Abstract**

The present invention relates to the use of compounds of general formula (I): 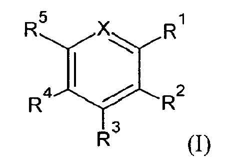 for the preparation of medicaments that act against mitochondrial pathologies involving a deficiency in ATP production via the oxidative phosphorylation pathway, such as mitochondrial diseases.

## Description

The present invention relates to the isolation and development of drugs to treat mitochondrial pathologies involving a deficiency in ATP production via the oxidative phosphorylation pathway, such as NARP syndrome.

NARP (Neuropathy, Ataxia and Retinitis Pigmentosa) is a maternally transmitted hereditary syndrome characterized by retarded development, and accompanied by retinitis pigmentosa (RP), dementia, ataxia, proximal neurological muscle weakness and sensory neuropathies (Schon et al., J. Bioenerg. Biomembr., 1994, 26, 291-299; Graeber, M.B. and Müller, U., J. Neurol. Sci., 1998, 153, 251-263, for review). This disease is in general a pathology which occurs in children, but it has also been reported in rarer cases in adults. The clinical manifestations are varied and can take more or less severe forms. Thus, the ophthalmic manifestations can range from a simple "salt and pepper" changing of the retina to severe RP, accompanied by maculopathy. Similarly, there is a broad spectrum of neurological manifestations, which ranges from simple migraines to severe dementia and to "Leigh's disease" (subacute necrotising encephalomyelopathy; Ortiz et al., Arch., Ophtalmol., 1993, 111, 1525-1530). Many retinitis pigmentosarelated syndromes exist, such as Usher's syndrome in which both the sight and the hearing are affected, or else macular dystrophy, also called inverse RP.

In 1990, Holt et al. (Am. J. Hum. Genet., 46, 428-433) described for the first time the presence of the T8993G mutation in the mitochondrial DNA of patients showing NARP syndrome/Leigh's disease. It was subsequently postulated by Tatuch and Robinson (Biochem. Biophys. Res. Commun., 1993, 192, 124-128) that this mutation -occurring in the ATP6 subunit- resulted in a reduction in ATP synthesis by impairing the mitochondrial ATP synthase complex. This mutation is thought to be responsible for an ATP synthase assembly/stability defect (Nijtmans et al., J. Biol. Chem., 2001, 276, 6755-6762). Other *ATP6* gene mutations have also been detected, in association with NARP syndrome/Leigh's disease; T8993C, T9176G, T9176C, T8851C, T9185C and T9191C (Schon et al., Cell & Dev. Biol., 2001, 12, 441-448 and Kucharczyk et al., Biochimica et Biophysica Acta, 2009, 1793, 186-199). In addition, a T9101C mutation has been involved in the LHON (*Leber's Hereditary Optic Neuropathy)* syndrome, another mitochondrial syndrome (Kucharczyk et al., Biochimica et Biophysica Acta, 2009, 1793, 186-199). Simple point mutations are therefore responsible for these syndromes, which have many more or less serious forms. The great diversity of the pathological manifestations is attributed to the heteroplasmic nature of this mutation in patients, i.e. the coexistence of mutated and wild-type mitochondrial DNA molecules in the cells or tissues. The mutated mitochondrial DNA load is closely correlated with the seriousness of the symptoms observed (Uziel et al., J. Neurol. Neurosurg. Psychiatry, 1997, 63, 16-22; Carelli et al., Arch. Neurol., 2002, 59, 264-270).

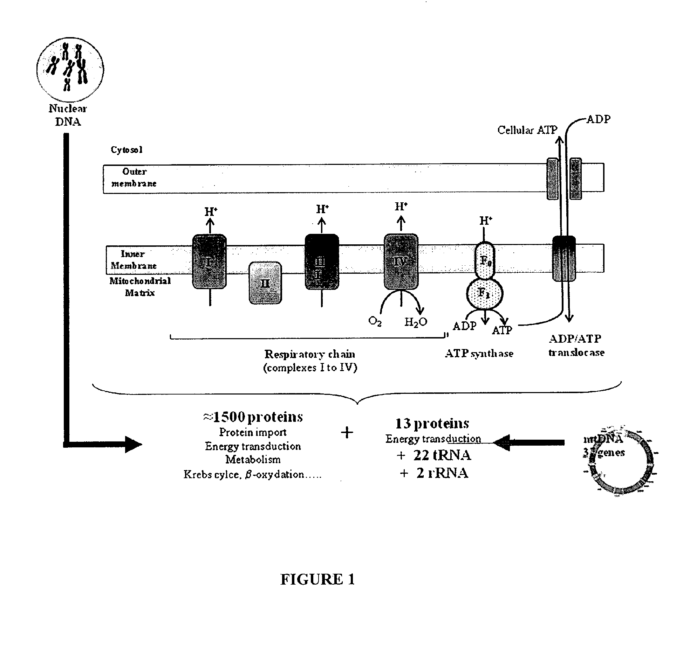

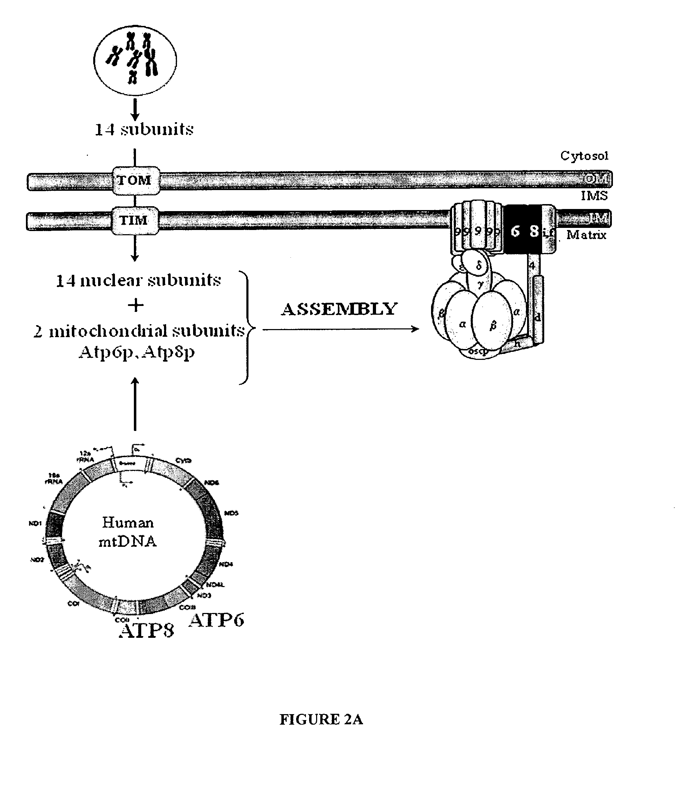

The ATP synthase complex, which is the target of the T8993G mutation (and of the other mutations mentioned above), is located in the inner mitochondrial membrane (Figures 1 and 2A). It catalyzes the last steps of oxidative phosphorylation, a process which allows cells to extract the chemical energy of metabolites and to store this energy in ATP molecules. In order to synthesize ATP, the ATP synthase complex uses the electrochemical proton gradient on either side of the inner membrane, generated by other complexes located in this membrane, the respiratory complexes (Figure 1). The latter transfer to oxygen the reducing equivalents of the substrates that are oxidized in the mitochondrion. These transfers are coupled to proton transports (hydrogen ions, H⁺) across the inner membrane, from the inside (the mitochondrial matrix) into the space between the outer and inner membranes (intermembrane space) of the organelle. The result is a proton concentration that is higher at the outer periphery of the inner membrane than at its inner periphery. The membrane domain Fₒ (Figure 1) of ATP synthase enables a channeled return of the protons into the mitochondrial matrix. This transport is coupled to ATP synthesis in the catalytic domain F₁ of ATP synthase located outside the membrane, in the mitochondrial matrix. ATP synthase operates like a rotary turbine: the passage of protons in Fₒ is coupled to the rotation of a subcomplex (the rotor) of the enzyme. This rotation results in conformational changes in F₁ which promote the synthesis of ATP from ADP and inorganic phosphate (Boyer P.D., Annu, Rev., Biochem., 1997, 66, 717-747). The neosynthesized ATP molecules can, via a specific transporter located in the inner membrane (ADP/ATP translocase), leave the mitochondrial compartment so as to supply the entire cell with energy. ATP synthase comprises about twenty different protein subunits for a mass of approximately 600 KDa. In humans, two ATP synthase subunits (Atp6p and Atp8p, Figure 2A) are encoded by the mitochondrial genome, all the other subunits being encoded by nuclear genes. The subunits of nuclear origin are synthesized in the cytosol and then imported into the mitochondrion, whereas the Atp6p and Atp8p subunits encoded by the mitochondrial genome are actually synthesized inside the mitochondrion (Figure 2A).

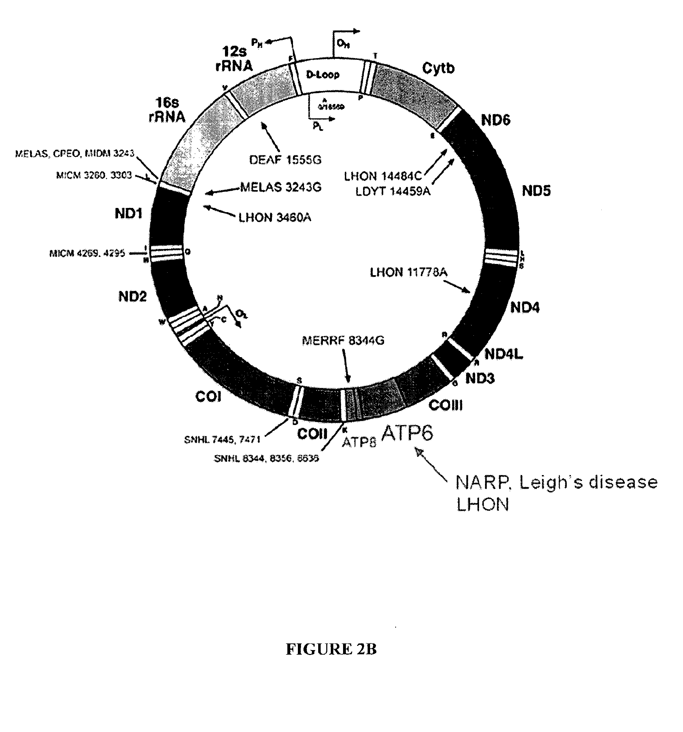

The T8993G mutation associated with NARP syndrome is located within the mitochondrial *ATP6* gene (Figure 2B). The latter encodes ATP synthase subunit 6 (Atp6p) which is essential for proton transport across Fₒ. The T8993G mutation results in the replacement, with arginine, of a leucine residue conserved in all the known sequences of Atp6p, from bacteria to humans. This leucine residue is in an Atp6p region presumed to be transmembrane and essential for ATP synthase proton translocation activity. Studies carried out in the *Escherischia coli* bacterium or with NARP cybrids (human cells in which the mitochondria are enriched, up to 100%, in T8993G alleles) indicate that the T8993G mutation clearly affects the functioning of the ATP synthase proton channel and that this defect is the primary cause of the disease (Schon et al., Cell & Dev. Biol., 2001, 12, 441-448; Nijtmans et al., J. Biol. Chem., 2001, 276, 6755-6762).

There is currently no effective medicament for the treatment of mitochondrial disorders induced by F₁f₀-ATP synthase dysfunctions.

A cellular model for the NARP syndrome has recently been developed consisting in yeast strains carrying within their mitochondrial genome, the equivalent of mitochondrial *ATP6* gene mutations responsible for NARP syndrome in humans (see the International PCT Application WO 2007/125225).

These yeast mutants make it possible to identify molecules capable of correcting the effects of the mutation by restoring either ATP synthase function, or sufficient production of ATP in the mitochondria, via a pathway other than that of oxidative phosphorylation.

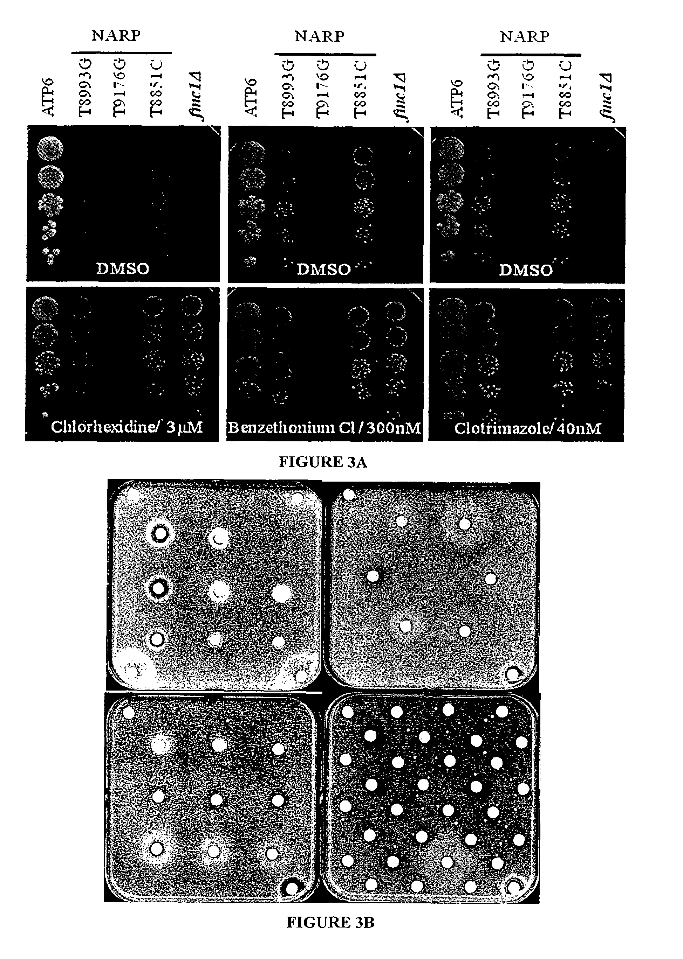

The invention concerns compounds which have been selected for their ability to restore respiratory growth of the yeast *ATP6* mutant (Figure 3A and 3B).

An object of the present invention concerns the use of compounds having the general formula (I): 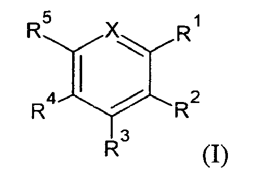 wherein
- X is a carbon atom or a nitrogen atom, both atoms being optionally substituted by an oxygen atom;
- **R¹** is a hydrogen atom, a halogen atom or a sulphur atom;
- **R²** is a hydrogen atom or
   **R¹** and **R²** taken together with the carbon atoms to which they are attached may form the following cycle 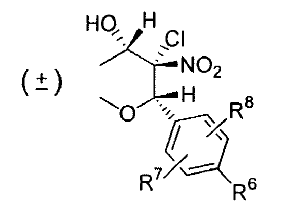 with the proviso that when **R¹** and **R²** form the above defined cycle, X is a carbon atom;
- **R³** is a hydrogen atom; a halogen atom; an alkyl radical containing 1 to 6 carbon atoms, preferably 1 to 3 carbon atoms, optionally interrupted by one oxygen atom or one ester function or an alkenylene radical containing 2 to 6 carbon atoms, preferably 2 to 3 carbon atoms,
   with the proviso that when **R¹** and **R²** do not correspond to the above defined cycle, **R³** is a hydrogen atom;
- **R⁴** is:
   - a hydrogen atom,
   - an alkyl radical containing 1 to 6 carbon atoms, preferably 1 to 3 carbon atoms, optionally interrupted by one oxygen atom,
   - a linear chain containing between 5 and 15 carbon atoms and between 1 and 10 nitrogen atoms,
      said chain optionally contains 1 to 5 oxygen atoms and is optionally interrupted by a benzyl group optionally substituted by one halogen atom; carbon and/or nitrogen atoms of said chain being optionally substituted by 1 or 2 alkyl radicals containing 1 to 3 carbon atoms, preferably a methyl radical, and carbon atoms of said chain being optionally substituted with =NH function;
   - the group 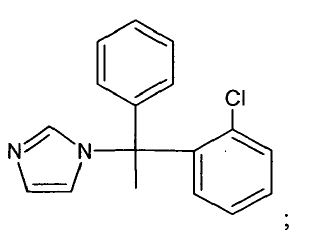
- **R⁵** is a hydrogen atom, a halogen atom, or a group 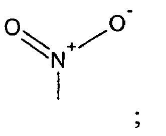 **R⁴** and **R⁵** taken together with the carbon atoms to which they are attached may form the following cycle 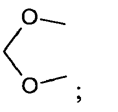
- **R⁶, R⁷** and **R⁸,** which may be identical or different, are:
   - a hydrogen atom;
   - a halogen atom;
   - an alkyl radical containing 1 to 6 carbon atoms, preferably 1 to 3 carbon atoms, optionally interrupted by one oxygen atom or one ester function and optionally containing a benzyl group;
   - a group -NH₂;
   - a group 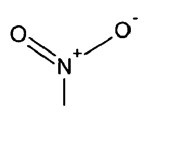 **R⁶** and **R⁸** taken together with the carbon atoms to which they are attached may form a benzyl group;
      with the proviso that
      when R¹ and R² correspond to the above defined cycle, R⁴ = R⁵ = R⁷ = R⁸ = H and R⁶ = Cl, R³ is not -O-CH₃ or
      when R¹ and R² correspond to the above defined cycle, R³ = R⁵ = R⁴ = R⁸ = H, and R⁷ is in ortho-position of R⁶, R⁶ and R⁷ are not simultaneously -O-CH₃,
      or a pharmaceutical acceptable salt
      for the preparation of a drug for the prevention and/or the treatment of disorders or diseases related to an insufficiency or a lack of ATP synthesis by F₁F₀-ATP synthase or related to an excessive accumulation of reactive oxygen species (ROS) chosen amongst mitochondrial diseases, normal or physiological ageing and neurodegenerative diseases.

By "halogen atom" is intended to mean fluorine, chlorine, bromine or iodine.

By "alkyl radical" containing 1 to 6 carbon atoms, is intended to mean a saturated, linear or branched, chain of 1 to 6 carbon atoms, such as methyl, ethyl, propyl, isopropyl, butyl, pentyl, hexyl...

By "alkenylene radical containing 2 to 6 carbon atoms", is intended to mean an unsaturated, linear or branched, chain of 2 to 6 carbon atoms containing one carbon-to-carbon double bond.

Examples of alkyl radicals containing 1 to 6 carbon atoms interrupted by one oxygen atom are of the general formula -(CH₂)ₙ-O-(CH₂)_{n'}-CH₃, n and n' being two integers comprised between 0 and 6 such as n + n' ≤ 6; an example of such radical is -O-CH₃.

Example of alkyl radicals containing 1 to 6 carbon atoms, preferably 1 to 3 carbon atoms, optionally interrupted by one oxygen atom or one ester function and optionally containing a benzyl group are: -O-CH₃, -O-CH₂-C₆H₆, -CO-O-CH₃, and -CO-O-CH₂-C₆H₆...

A non-limitating example of linear chain containing between 5 and 15 carbon atoms and between 1 and 10 nitrogen atoms; said chain being optionally interrupted by a benzyl group optionnally substituted by one halogen atom; carbon atoms of said chain being optionally substituted with =NH function is the following: - NH-C(NH)-NH-C(NH)-NH-(CH₂)₆-NH-C(NH)-NH-C(NH)-NH-C₆H₄-Cl.

A non-limitating example of linear chain containing between 5 and 15 carbon atoms and between 1 and 10 nitrogen atoms, said chain optionally containing 1 to 5 oxygen atoms and being optionally interrupted by a benzyl group; carbon and/or nitrogen atoms of said chain being optionally substituted by 1 or 2 alkyl radicals containing 1 to 3 carbon atoms, preferably a methyl radical; is the following: - CH₂-NH₂-(CH₂)₂-O-(CH₂)₂-O-C₆H₄-C(CH₃)₂-CH₂-C(CH₃)₂-CH₃.

In a first mode of carrying out the invention, compounds of formula (I) are such as R¹ en R² do not correspond to the above-defined cycle; then preferred compounds are selected in the following compounds :
Compound of Formula A. Chlorhexidine 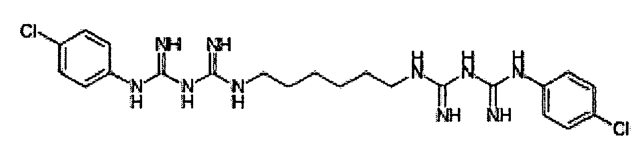 wherein X = C, R¹ = -Cl, R² = R³ = R⁵ = -H and R⁴ = -NH-C(NH)-NH-C(NH)-NH-(CH₂)₆-NH-C(NH)-NH-C(NH)-NH-C₆H₆-Cl
Compound of Formula B. Benzethonium chloride 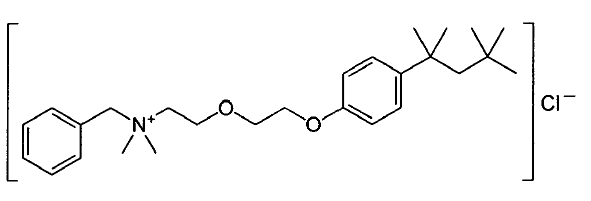 wherein X = C, R¹ = R² = R³ = R⁵ = -H and R⁴ = -CH₂-NH₂-(CH₂)₂-O-(CH₂)₂-O-C₆H₆-C(CH₃)₂-CH₂-C(CH₃)₂-CH₃
Compound of Formula C. Clotrimazole 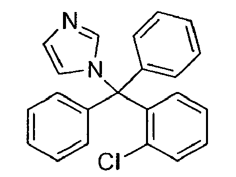 wherein X = C, R¹ = R² = R³ = R⁵ = -H
   and 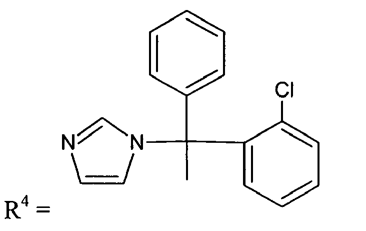
Compound of Formula D. Sodium pyrithione 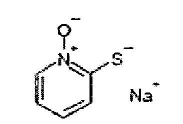 wherein X = N⁺-O⁻, R¹ = S and R² = R³ = R⁴ = R⁵ = -H

Chlorhexidine is known to have antiseptic properties, it shows also bactericidal properties, it kills both gram-positive and gram-negative bacteria.

Benzethonium chloride is a synthetic quaternary ammonium salt with surfactant, antiseptic and anti infective properties.

Clotrimazole is an antifungal agent.

Sodium pyrithione (CAS Registry number: 15922-78-8 ; 3811-73-2) is a large spectrum antimicrobial agent; it inhibits the growth of fungi, yeast, mold and bacteria.

Those four compounds are commercially available; their syntheses are reported in the literature: chlorexidine may be prepared according to US Patent 2,684,924; benzethonium chloride may be prepared according to US Patents 2,115,250, 2,170,111 and 2,229,024; clotrimazole may be prepared according to South African Patents 68 05,392 and 69 00,039 (Bayer) and sodium pyrithione may be prepared according to Shaw et al. J. Amer. Chem Soc. 72, 4362 (1950) or to US Patent 2,745,826.

In another mode of carrying out the invention, compounds of formula (I) are such as R¹ and R² form the above-defined cycle; the preferred compounds are those defined by the general formula (Ia): 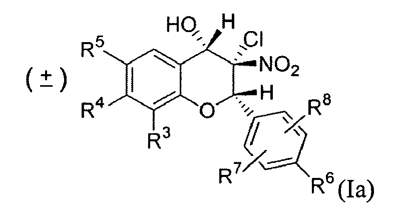 wherein **R³, R⁵, R⁶, R⁷** and **R⁸** are defined as above;
- **R⁴** is:
   - a hydrogen atom;
   - an alkyl radical containing 1 to 6 carbon atoms, preferably 1 to 3 carbon atoms, optionally interrupted by one oxygen atom; or
      **R⁴** and **R⁵** taken together with the carbon atoms to which they are attached may form
      the cycle 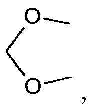 or a pharmaceutical acceptable salt of compounds of general formula (Ia).

According to a particular object, the invention relates to compounds of general formula (Ia) as drug, particularly, as active agents.

The following compounds of formula (Ia) are preferred:
Compound of Formula 1. (ICC005-L-001-A11) 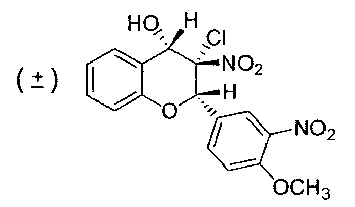 Compound of Formula 2. (ICC005-M204-C05) 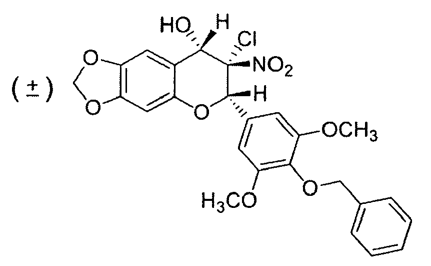
Compound of Formula 3. (ICC005-L006-D11) 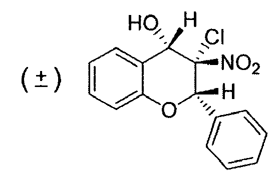 Compound of Formula 4. (ICC005-L028-D02) 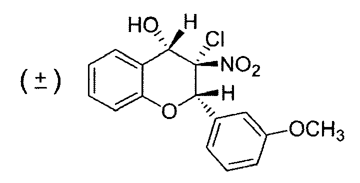 Compound of Formula 5. (ICC005-L025-A02) 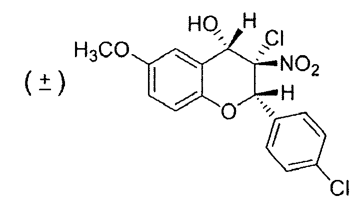
Compound of Formula 6. (ICC005-L023-B02) 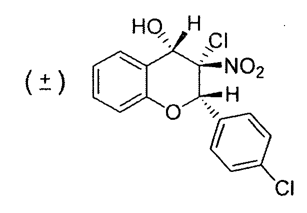
Compound of Formula 7. (ICC005-L040-D06) 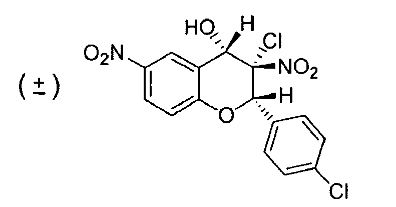
Compound of Formula 8. (ICC005-L134-H07) 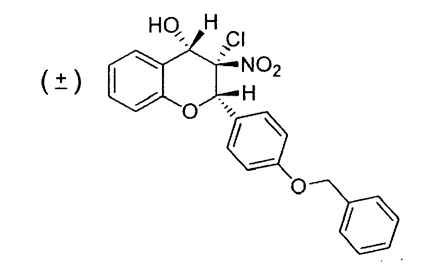
Compound of Formula 9. (ICC005-L022-B05) 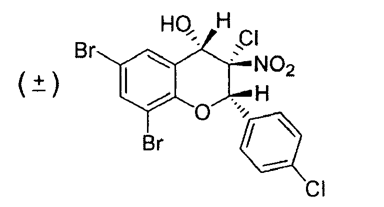
Compound of Formula 10. (ICC005-L040-E11) 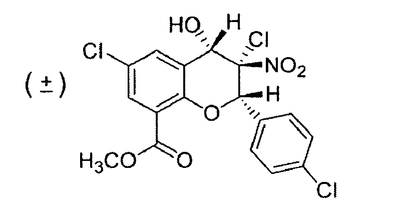
Compound of Formula 11. (ICC005-L037-H11) 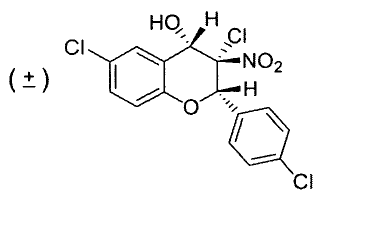
Compound of Formula 12. (ICC005-L037-G07) 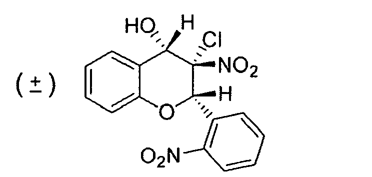
Compound of Formula 13. (ICC005-L036-A07) 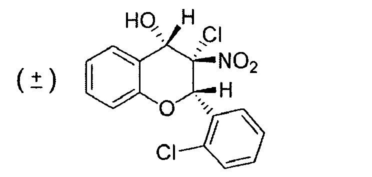
Compound of Formula 14. (ICC005-L024-E06) 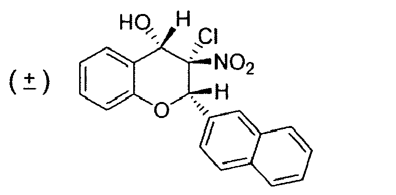
Compound of Formula 15. (ICC005-L032-H02) 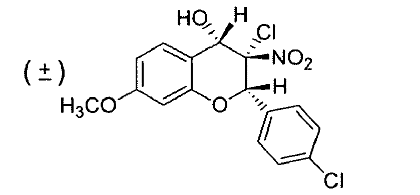
Compound of Formula 16. (ICC005-L034-E11) 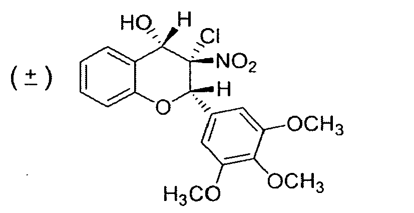
Compound of Formula 17. (ICC005-L033-E02) 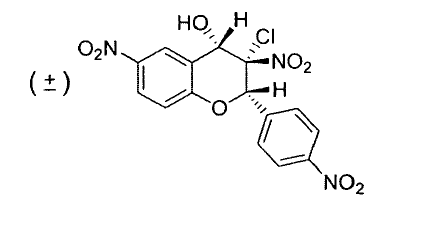
Compound of Formula 18. (ICC005-L018-C11) 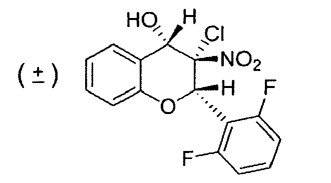
Compound of Formula 19. (ICC005-L028-E06) 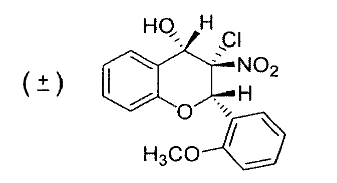
Compound of Formula 20. (ICC005-L018-E05) 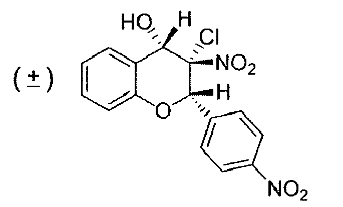
Compound of Formula 21. (ICC005-L002-D06) 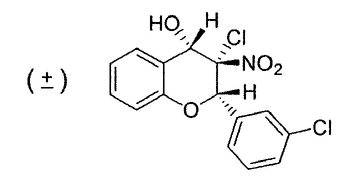
Compound of Formula 22. (ICC005-L002-F04) 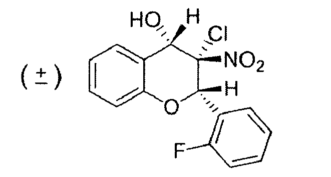
Compound of Formula 23. (ICC005-L002-C03) 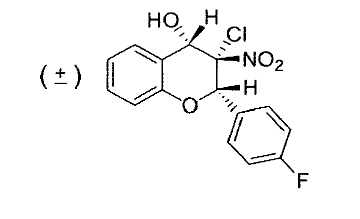
Compound of Formula 24. (ICC005-L002-G02) 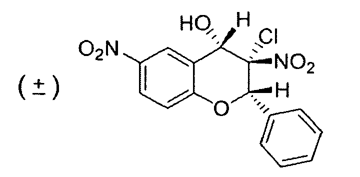
Compound of Formula 25. (ICC005-L019-B10) 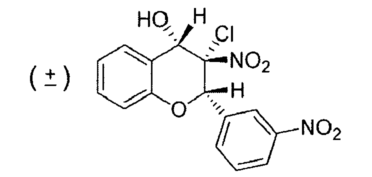
Compound of Formula 26. (ICC005-L001-B11) 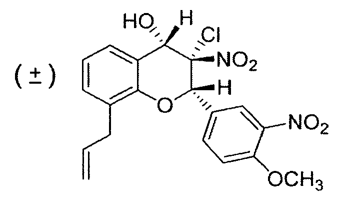
Compound of Formula 27. (ICC005-L015-E03) 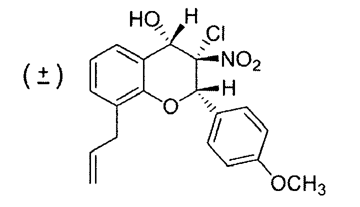
Compound of Formula 28. (ICC005-M204-C06) 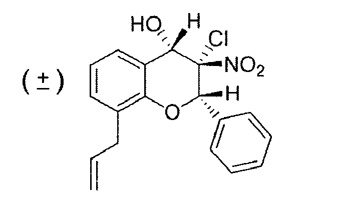
Compound of Formula 29. (ICC005-L145-E05) 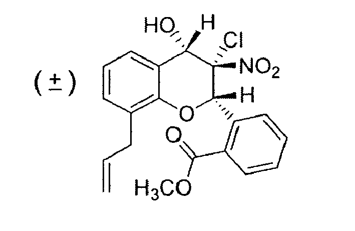
Compound of Formula 30. (ICC005-L145-E11) 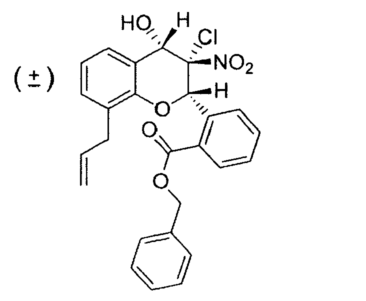
Compound of Formula 31. (ICC005-L145-H02) 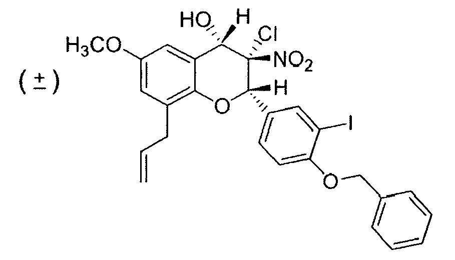
Compound of Formula 32. (ICC005-L145-H06) 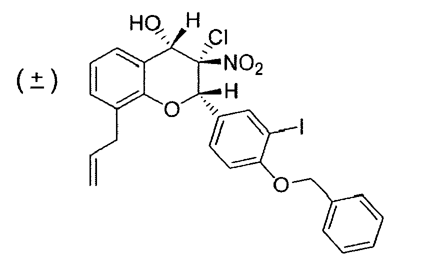
Compound of Formula 33. (ICC005-L046-F07) 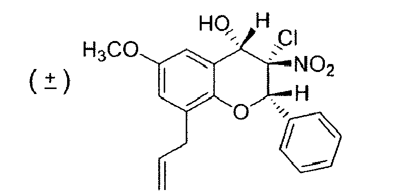
Compound of Formula 34. (ICC005-L044-H06) 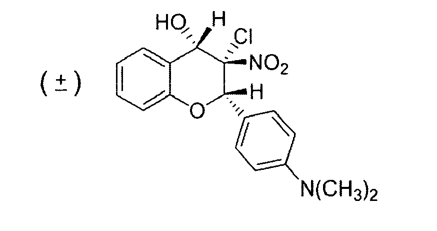
Compound of Formula 35. (ICC005-L046-C11) 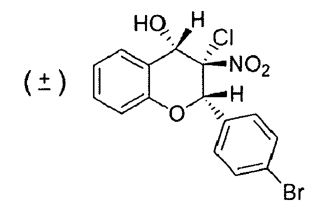
Compound of Formula 36. (ICC005-L045-E06)

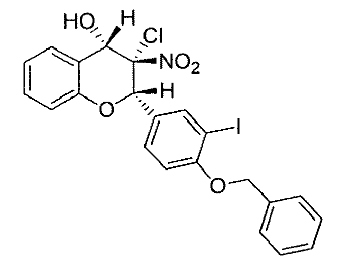

The synthesis of compounds of general formula (Ia) is described below.

Compounds of general formula (Ia) may be prepared by the condensation of a salicylic aldehyde 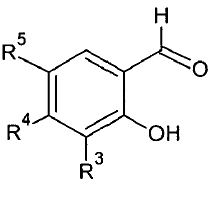 and a (*Z*) β-chloro-β-nitrostyrene 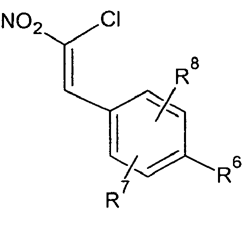 with triethylamine in THF in strictly anhydrous conditions.

More specifically,
- Compounds of Formula 3, 4, 5, 6, 7, 9, 11, 12, 13, 14, 15, 16, 19, 20, 21 and 25 may be prepared according to the article of D. DAUZONNE et al. (Synthesis, 66-70 (1990));
- Compounds of Formula 1, 17, 18, 22, 23, 24 and 28 may be prepared according to the article of D. DAUZONNE et al. (Eur. J. Med. Chem., 32, 71-82 (1997));
- Compounds of Formula 2 may be prepared according to the article of A. GONZALEZ DE PEREDO et al. (Chem. Pharm. Bull. 46, 79-83 (1998));
- Compounds of Formula 26, 27, 29 and 30 may be prepared according to the article of B. BAUVOIS et al. (J. Med. Chem. 46, 3900-3913 (2003)).

Another object of the present invention relates to compounds of Formula 8, 10, 31, 32, 33, 34, 35 and 36.

The hitherto unknown compounds 8, 10, 31, 32, 33, 34, 35 and 36 may be prepared, starting from the appropriate salicylaldehydes and (*Z*) b-chloro-b-nitrostyrenes, according to the methodology reported in the article of D. DAUZONNE et P. DEMERSEMAN (Synthesis, 66-70 (1990)).

According to the present invention, preferred compounds of general formula (Ia) are compounds 5, 6, 7, 8, 9, 10, 11, 12, 13, 14, 15, 18, 20, 21, 22, 23, 24, 25, 27, 32 and 36.

More preferred compounds of general formula (Ia) are compounds 5,6,7,8,10,11,13, 15, 18, 21, 22,23, 32 and 36.

The biological activities of the compounds of general formula (I) have been evaluated.

The ability of compounds of general formula (I) to restore respiratory growth of the yeast mutant has been evaluated with a yeast strain bearing a mutation on *ATP6* (Figure 3 and Example 1) and also with a yeast *fmc1*Δ mutant which affect the assembly of the ATPase complex.

These properties make said compounds as well as their salts suitable for use as drugs in the treatment of disorders or diseases linked with alteration of the F₁F₀-ATP synthase pathway; such diseases or disorders are those resulting from an insufficiency or a lack of ATP synthesis by F₁F₀-ATP synthase; and those related to an excessive accumulation of reactive oxygen species (ROS) which results from insufficiency or lack of ATP synthesis.

The use of the compounds of general formula (I) leads to an increase in ATP production and/or a decrease in ROS accumulation in cells.

As a consequence, compound of general formula (I) are useful for the preparation of a drug for preventing and/or treating mitochondrial diseases, in particular for the treatment of mammals, such as human.

As already explained, mitochondrial diseases often result from a deficiency in ATP production -via the oxidative phosphorylation- which makes high energy-demanding tissues or organs such as heart, brain, and muscles, the main targets for these disorders.

Other mitochondrial alterations also contribute to pathologies amongst which an increased production of reactive oxygen species (ROS), responsible for important cellular oxidative damages, a low rate of NADH reoxidation and defective calcium storage within the organelle; examples of these pathologies are the syndromes NARP, LHON, MILS (*Maternally Inherited Leigh Syndrome*), MERRF (*Myoclonic Epilepsy with Ragged-Red Fibers*) and HSP (*Hereditary Spastic Paraplegia*).

Compounds of general formula (I) appear to be also useful for the preparation of a drug for preventing and/or treating an acceleration of normal or physiological ageing. Physiological ageing is characterized by numerous phenomena; it may be characterized by the decrease of cells renewal; by the decrease of cells life and/or by the decrease of the number of cells in an organ, leading to atrophy and sometimes to dysfunction of said organ. Other evidences of ageing are modification of the appearance such as loss and/or graying of the hair, modification of appearance of the skin...

As mitochondrial disorders are observed during the progress of neurodegenerative diseases, the present invention also relates to the use of compounds of general formula (I) for preventing and/or treating neurodegenerative diseases such as Alzheimer's disease, myasteny disease, Parkinson's disease...

In addition to the above provisions, the invention also comprises other provisions which will become clear from the description which follows, which refers to examples illustrating the biological activity of compounds of general formula (I), and also to the attached drawings in which:
**Figure 1** is a schematic representation of the mitochondrial energy transduction apparatus and of the genes controlling the formation thereof.
**Figures 2A** **and** **2B** illustrate the structure of the ATP synthase and the genes encoding this protein complex: the ATP synthase is composed of subunits which are encoded by the nuclear DNA and by the mitochondrial DNA (Atp6p, Atp8p) and assembled in the mitochondrial matrix. Figure 2B focuses on the mitochondrial DNA and the mutations which are associated with human mitochondrial disease (from mitomap.org).
**Figure 3A and 3B** are pictures of Petri dishes showing the ability of compounds of general formula (I) to restore respiratory growth of the ATP synthase yeast mutant on nonfermentable medium. The three upper panels of figure 3A show that the ATP synthase mutants strains (three NARP strains and *the fmc1Δ*) grow very slowly on a respiratory medium (nonfermentable carbon source) due to a defect of the ATP synthase (Example 1).

The three lower panels of Figure 3A illustrate the improvement of the respiratory growth when compounds (Chlorhexidine, Benzethonium Chloride or Clotrimazole instead of DMSO, the negative control) are added into the agar medium at different concentrations.

On Figure 3B, the mutant cells are plated out in a layer at the surface of an agar medium containing nonfermentable carbon source and compounds are added on filters and disposed on Petri dish. The negative control, DMSO and the positive control, oleate, are loadded on the upper left filter and on the lower right filter respectively.

**Figures 4 to 8** are graphs showing the effect of compounds of general formula (I) on life expectancy of *Drosophila* having mitochondrial encephalomyopathy (Example 2).

### Example 1: Demonstration of the capacity of compounds of general formula (I) to restore respiratory growth of mutant yeasts on nonfermentable medium

The *ATP6* yeast mutants grow very slowly from a nonfermentable carbon source due to a dysfunction of the ATP synthase. These yeast mutants are therefore used to identify molecules capable of correcting the effects of the mutation by restoring either ATP synthase function, or ATP production by the mitochondria allowing the yeasts to grow on nonfermentable medium.

Mutant strains used in this experiment are:
- MC6 (*fmc1Δ*) (Lefebvre-Legendre L. et al., J. Biol. Chem., 2001, Mar2, 276 (9) : 6789-96)
   Genotype: MC6: *Mat* α, *ade2, leu2, ura3, trp1, his3, fmc1::HIS3*
- MR14 (NARP T8993G) which may be obtained according to International PCT Application WO 2007/125225.
   Genotype: MR14: *Mat a, ade2, leu2, ura3, trpl, his3, arg8::HIS3[rho⁺ FY1679; atp6 T8993G]*

The principle of the activity test is the following:
Step 1: the mutant yeast is cultured in medium containing glucose (YPAD medium).
Step 2: the mutant cells are plated out in a layer at the surface of an agar medium containing a nonfermentable carbon source such as glycerol (YPG medium).
Step 3: filters which each contain a defined amount of one of the test molecules are placed on the Petri dish, the molecules diffuse in the medium and establish a concentration gradient around the filters.
Step 4: the dishes are incubated at 35 or 36°C.

Under these conditions, a growth halo is seen to appear around the filters containing a substance capable of counteracting the effects of the mutation.

### Media

YPG medium contains 1% Yeast Extract; 2% Bactopeptone; 2% Bactoagar only for solid medium; 2% glycerol (expressed in weight/volume); Adenine 60mg/lL and spenicilline 30 000UI/L.

Liquid YPAD medium contains 1% Yeast Extract; 2% Bactopeptone; 2% glucose (expressed in weight/volume) and Adenine 60mg/L.

### The day before

For each of the MC6 and MR14 strains, one colony from a YPAD agar plate has been inoculated in 4 ml of liquid YPAD medium.

### The day of assay

In the morning, the optical density (OD) of the culture is measured; the culture is diluted in liquid YPAD medium (50 or 100 µl in 4ml of medium if the broth is saturated).

After 4-5 h, OD of the culture is measured; the culture is diluted in YPG medium until the OD is 0.2.

240 µl of the culture is introduced in squared dishes (12cm /12cm) and plated with sterile glass beads.

Sterile filters are introduced into the dishes.

Then filters are impregnated with either:
- 1 µl/filter of positive control (oleate 100 mM);
- 1 µl/filter of negative control (DMSO, compounds vehicle, pure from Sigma);
- 2.5 µl/filter of compounds having general formula (Ia) 10 mg/ml in DMSO;
- 2.5 µl/filter of compounds A, B, C and D 5 mg/ml in DMSO.

The dishes are incubated at 35°C for MR14 yeast strain or 36°C for MC6 yeast strain.

### Results

Results for chlorhexidine, benzethonium chloride or clotrimazole are illustrated in **Figures 3A****.**

Activity of the compounds is detected when mutant yeasts grow around the filters forming a growth halo; the size and the density of said halo allow defining a qualitative value for activity. All the selected drugs are active on both mutant strains (MR14 and *fmc1Δ*).

| **Compounds** | **activity** |
|---|---|
| 5 | ++ |
| 6 | ++ |
| 7 | ++ |
| 8 | +++ |
| 9 | + |
| 10 | ++ |
| 11 | ++ |
| 12 | + |
| 13 | ++ |
| 14 | + |
| 15 | ++ |
| 18 | ++ |
| 20 | + |
| 21 | ++ |
| 22 | ++ |
| 23 | ++ |
| 24 | + |
| 25 | + |
| 27 | + |
| 32 | ++ |
| 36 | ++ |
| A- Chlorhexidine | +++ |
| B- Benzethonium | ++ |
| C- Clotrimazole | +++ |
| D- Sodium Pyrithione | ++ |

### Example 2 - Demonstration of the capacity of compounds of general formula (I) to improve lifespan of Drosophila suffering from mitochondrial encephalomyopathy

Compounds of general formula (I) have then been tested on a *Drosophila* model described by Celotto et al. (Mitochondrial Encephalomyopathy in Drosophila, The Journal of Neuroscience, January 18, 2006 - 26(3):810-820). These Drosophila mutants are known to show a shorter lifespan.

### Methods for Drosophila Drug Screen

mtATP6[1];sesB[1]/sesB[1] flies were outcrossed once to produce females with the genotype mtATP6[1];sesB[1]/+ for study.

Each drug was dissolved in 0.09% DMSO to the following final concentrations: 15 µM, 1 µM, 50 nM and 2.5 nM.

Approximately 20 flies were tested per vial and 3 independent vials were tested for each concentration of each drug.

Newly eclosed females were counted and placed into a vial with approximately 10 milliliters standard cornmeal molasses media. Test compounds were applied (25 microliters at the specified concentrations) to a semi-circle of filter paper covering about 1/2 of the surface of the media.

Longevity experiments were performed using 12:12 light dark regime at 25 C.

Flies were counted every other day at which time food, filter paper and drug were replaced until all flies had expired. Survival curves were generated and analyzed using Prism 4.0b and log rank tests were performed to determine significance from the vehicle only controls.

### Results

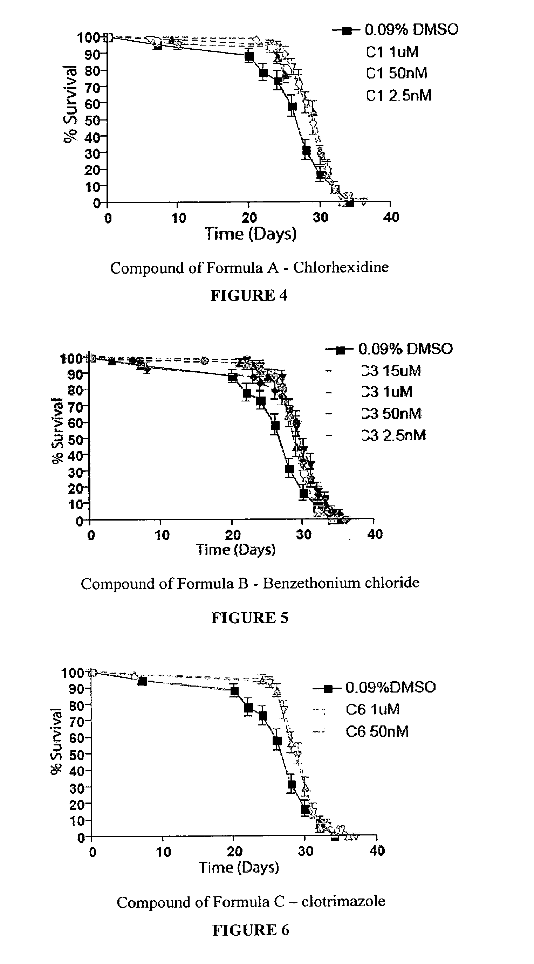

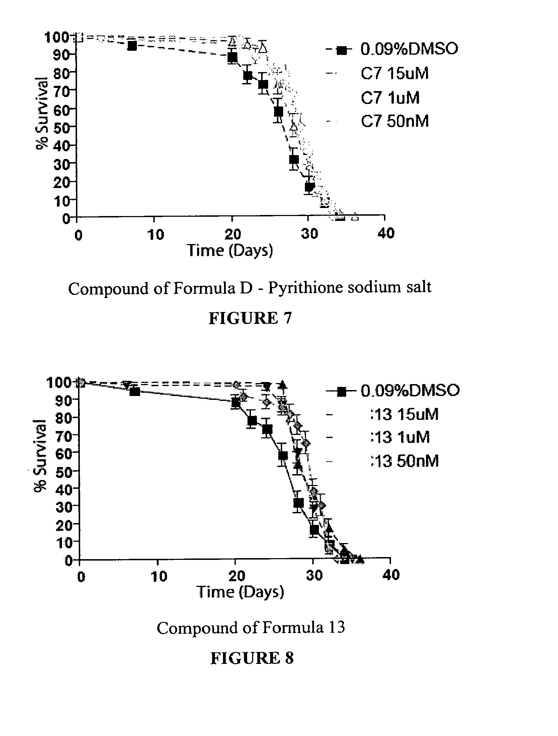

Graphs of Figures 4 to 8 show a significant increase in Drosophila's life after treatment with Compound of Formula A - Chlorhexidine (**Figure 4**), Compound of Formula B - Benzethonium chloride (**Figure 5**), Compound of Formula C - clotrimazole (**Figure 6**), Compound of Formula D - Pyrithione sodium salt (**Figure 7**) and Compound of Formula 13 (**Figure 8**).

## Claims

1. Use of the compounds having the general formula (I): 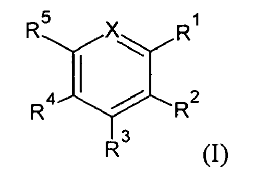 wherein
- **X** is a carbon atom or a nitrogen atom, both atoms being optionally substituted by an oxygen atom;
- **R¹** is a hydrogen atom, a halogen atom or a sulphur atom;
- **R²** is a hydrogen atom or
**R¹** and **R²** taken together with the carbon atoms to which they are attached may form
the following cycle 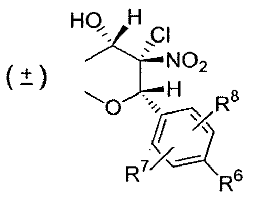 with the proviso that when **R¹** and **R²** form the above defined cycle, X is a carbon atom;
- **R³** is a hydrogen atom; a halogen atom; an alkyl radical containing 1 to 6 carbon atoms, preferably 1 to 3 carbon atoms, optionally interrupted by one oxygen atom or one ester function or an alkenylene radical containing 2 to 6 carbon atoms, preferably 2 to 3 carbon atoms,
with the proviso that when **R¹** and **R²** do not correspond to the above defined cycle, **R³** is a hydrogen atom;
- **R⁴** is:
- a hydrogen atom,
- an alkyl radical containing 1 to 6 carbon atoms, preferably 1 to 3 carbon atoms, optionally interrupted by one oxygen atom,
- a linear chain containing between 5 and 15 carbon atoms and between 1 and 10 nitrogen atoms,
said chain optionally contains 1 to 5 oxygen atoms and is optionally interrupted by a benzyl group optionally substituted by one halogen atom; carbon and/or nitrogen atoms of said chain being optionally substituted by 1 or 2 alkyl radicals containing 1 to 3 carbon atoms, preferably a methyl radical, and carbon atoms of said chain being optionally substituted with =NH function;
- the group 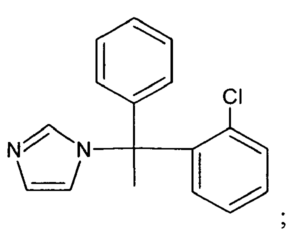
- **R⁵** is a hydrogen atom, a halogen atom, or a group 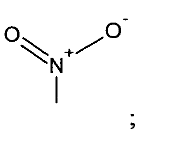 **R⁴** and **R⁵** taken together with the carbon atoms to which they are attached may form
the following cycle 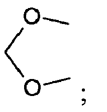
- **R⁶, R⁷** and **R⁸,** which may be identical or different, are:
- a hydrogen atom;
- a halogen atom;
- an alkyl radical containing 1 to 6 carbon atoms, preferably 1 to 3 carbon atoms, optionally interrupted by one oxygen atom or one ester function and optionally containing a benzyl group;
- - a group -NH₂;
- a group 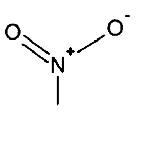 **R⁶** and **R⁸** taken together with the carbon atoms to which they are attached may form a benzyl group;
with the proviso that
when R¹ and R² correspond to the above defined cycle, R⁴ = R⁵ = R⁷ = R⁸ = H and R⁶ = Cl, R³ is not -O-CH₃ or
when R¹ and R² correspond to the above defined cycle, R³ = R⁵ = R⁴ = R⁸ = H, and R⁷ is in ortho-position of R⁶, R⁶ and R⁷ are not simultaneously -O-CH₃,
or a pharmaceutical acceptable salt
for the preparation of a drug for the prevention and/or the treatment of disorders or diseases related to an insufficiency or a lack of ATP synthesis or related to an excessive accumulation of reactive oxygen species (ROS).

2. Use according to claim 1, wherein said compounds are chosen amongst: compounds A, B, C and D.

3. Use according to claim 1, **characterized in that** said compounds are defined by the general formula (Ia): 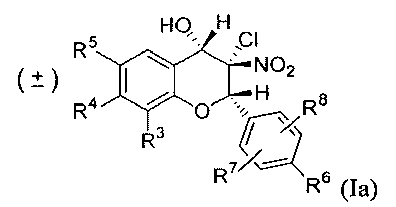 wherein **R³, R⁵, R⁶, R⁷** and **R⁸** are defined as above;
- **R**⁴ is:
- a hydrogen atom;
- an alkyl radical containing 1 to 6 carbon atoms, preferably 1 to 3 carbon atoms, optionally interrupted by one oxygen atom; or
**R⁴** and **R⁵** taken together with the carbon atoms to which they are attached may form the cycle 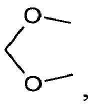 or pharmaceutical acceptable salt of compounds of general formula (Ia).

4. Use according to claim 3, wherein said compounds are chosen amongst: 5, 6, 7, 8, 9, 10, 11, 12, 13, 14, 15, 18, 20, 21, 22, 23, 24, 25, 27, 32 and 36.

5. Use according to anyone of claims 1 to 4, wherein said disorders or diseases are mitochondrial diseases.

6. Use according to claim 4, wherein said mitochondrial diseases are the syndromes NARP, LHON, MILS, MERRF and HSP.

7. Use according to anyone of claims 1 to 4, wherein said compounds are used for the preparation of drug for preventing and/or treating an acceleration of physiological ageing.

8. Use according to anyone of claims 1 to 4, wherein said compounds are used for the preparation of drug for preventing and/or treating neurodegenerative diseases such as Alzheimer's disease, myasteny disease, Parkinson's disease.

9. Compounds of general formula (Ia) as defined above as drug.

10. Compounds of Formula 8, 10, 31, 32, 33, 34, 35 and 36.
